**Europäisches Patentamt**

**European Patent Office**

**Office européen des brevets**

(19)

(11) Veröffentlichungsnummer: **0 235 691 B1**

(12) # EUROPÄISCHE PATENTSCHRIFT

(45) Veröffentlichungstag der Patentschrift:
20.03.91 Patentblatt 91/12

(51) Int. Cl.⁵: **A61J 1/06, A61M 5/31**

(21) Anmeldenummer: **87102367.7**

(22) Anmeldetag: **19.02.87**

---

(54) **Vorrichtung zur Applikation von Arzneimittelsuspensionen.**

---

Teilanmeldung 90113525.1 eingereicht am 19/02/87.

(30) Priorität: **26.02.86 DE 3606163**

(43) Veröffentlichungstag der Anmeldung:
**09.09.87 Patentblatt 87/37**

(45) Bekanntmachung des Hinweises auf die Patenterteilung:
**20.03.91 Patentblatt 91/12**

(84) Benannte Vertragsstaaten:
**AT BE CH DE ES FR GB GR IT LI LU NL SE**

(56) Entgegenhaltungen:
**US-A- 2 688 966**
**US-A- 3 045 494**
**US-A- 3 187 749**
**US-A- 3 604 417**

(73) Patentinhaber: **HOECHST AKTIENGESELLSCHAFT**
**Postfach 80 03 20**
**W-6230 Frankfurt am Main 80 (DE)**

(72) Erfinder: **Grau, Ulrich, Dr.**
**Am Forsthaus 1**
**W-6238 Hofheim am Taunus (DE)**
Erfinder: **Pohler, Wolfgang, Dr.**
**Martin-Wohmann-Strasse 27**
**W-6238 Hofheim am Taunus (DE)**

EP 0 235 691 B1

Jouve, 18, rue Saint-Denis, 75001 PARIS

## Beschreibung

Bekannt sind tragbare Infusionsgeräte zur automatischen Abgabe von Flüssigkeiten, insbesondere Insulin, wobei diese Flüssigkeiten in auswechselbaren Spritzampullen enthalten sind (Europäische Patentanmeldung mit der Veröffentlichungsnummer 0 143 895 und darin genannte Vorrichtungen). Die US 2688966 beschreibt kompartimentierte Ampullen, wobei ein Kompartiment ein Medikament in trockener Form enthält und ein zweites, von dem ersten durch einen Stopfen getrenntes Kompartiment, das Lösungsmittel für das trockene Medikament enthält. Der zwischen den Kompartimenten befindliche Stopfen ist durchbohrt und enthält eine Kugel, die vor Benutzung der Vorrichtung aus diesem herausgedrückt wird. Auch für das Lösungsmittelkompartiment ist eine Kugel vorgesehen, die eventuell vorhandene Präzipitate wieder in Lösung bringen soll. Bekannt sind weiterhin tragbare Mehrfach-Injektionsgeräte, sogenannte "Pens"" (EP-A- 0 058 536, WO 82/02662). Diese bekannten Vorrichtungen, die zur Mehrfachapplikation der Arzneimittel bestimmt sind, eignen sich jedoch nur für homogene Flüssigkeiten, insbesondere Lösungen. Bei dispersen Systemen wie Arzneimittelsuspensionen besteht nicht nur die Gefahr, daß es durch Absetzen der Kristalle zu mechanischen Schwierigkeiten kommt, sondern vor allem zu Fehldosierungen. Solche Fehldosierungen sind besonders dann kritisch, wenn es sich bei dem Medikament um ein hochwirksames Arzneimittel von geringer therapeutischer Breite handelt, wie beispielsweise Insulin.

Werden bei einer Insulinsuspension zu wenig Kristalle appliziert, weil die Suspension ungenügend aufgeschüttelt bzw. bereits wieder sedimentiert war, so ist eine Hyperglykämie mit den bekannten begleitenden Symptomen und mit erhöhtem Risiko zu Spätkomplikationen zu erwarten. Wird umgekehrt zu viel des Wirkstoffs appliziert, so ist eine Hyperinsulinämie und eine Hypoglykämie mit der begleitenden schweren Symptomatik, wie Schweißausbrüche, Krämpfen Bewußtlosigkeit bis hin zum Tod durch hyperglykämischen Schock zu befürchten.

Arzneimittelsuspensionen sind normalerweise in Behältnisse abgefüllt, die über der Suspension auch einen Gasraum enthalten. Die Suspension läßt sich dann dadurch homogenisieren, daß dieses Behältnis gerollt, auf den Kopf gedreht oder geschüttelt wird. Dafür verantwortlich ist die Präsenz der Gasblase und deren Beweglichkeit. Ist dagegen die Suspension im wesentlichen gasraumfrei abgefüllt, beispielsweise in Zylinderampullen, aus denen mehrfach appliziert werden soll, ist eine homogene Vermischung in angemessener Zeit normalerweise nicht möglich. Die physikalische Grundlage für dieses Phänomen liegt darin, daß die Dichte von Kristallen im allgemeinen sehr ähnlich der Dichte des Mediums ist.

Bei Dosiervorrichtungen, in denen aus einem Vorrat mehrfach Dosierungen appliziert werden sollen, ist jedoch eine im wesentlichen gasblasenfreie Abfüllung notwendig, da nur so eine reproduzierbare Dosierung gewährleistet ist. "Im wesentlichen gasblasenfrei" soll hierbei nicht ausschliessen, daß anfangs, also beispielsweise in der Ausgangsampulle, eine mehr oder weniger große Gasmenge vorhanden ist, die aber vor der ersten Applikation in bekannter Weise entfernt wird, so daß dann die Flüssigkeit praktisch gasblasenfrei ist. Gasblasenfreie Kristallsuspensionen können jedoch nicht durch Schütteln in einem vertretbaren Zeitraum in die nötige homogene Form gebracht werden. Hier ist insbesondere zu berücksichtigen, daß eine solche Homogenisierung auch alten und behinderten Patienten möglich sein muß. Aufgabe der Erfindung war es daher, für Infusions- bzw. Injektionsgeräte geeignete Vorratsbehältnisse zur Verfügung zu stellen, in denen Arzneimittelsuspensionen auch in Abwesenheit einer Gasblase homogen aufgeschüttelt werden können.

Erschwerend kommt hinzu, daß die für eine parenterale Applikation notwendige Sterilität des Inhalts zu gewährleisten ist. Das Vorratsbehältnis muß also so beschaffen sein, daß es in gefülltem Zustand mit Hilfe der üblichen Verfahren sterilisiert oder nach Sterilisation der Einzelteile unter aseptischen Bedingungen montiert, mit steriler Arzneimittelsuspension befüllt und verschlossen werden kann.

Es ist bekannt, daß Schmiermittel-Disepersionen und Lacke, die in Sprühdosen abgefüllt sind, als Mischkörper Stahlkugeln enthalten. Bei den Schmiermitteln handelt es sich jedoch um leicht dispergierbare Stoffe wie Molybdänsulfid und Graphit, die durch wenige Schüttelbewegungen leicht in eine sprühfähige Form gebracht werden können. Bei Lacken liegt demgegenüber ein relativ viskoses System vor, so daß beim Schütteln hohe Scherkräfte auftreten, die die Redispergierung erleichtern. Im übrigen enthalten diese Systeme große Mengen Treibgas, so daß diese Art der Durchmischung nicht ohne weiteres auf Arzneimittelsuspensionen übertragbar ist. Außerdem entsteht durch das Reiben der Stahlkugeln an der Wand des Behälters ein Metallabrieb, der jedoch bei diesen technischen Systemen nicht stört. Es besteht auch nicht die Gefahr einer Rekristallisation und damit einer Partikelvergröberung, da die dispergierten Schmierstoffe oder Pigmentpartikel im Applikationsmedium völlig oder doch praktisch unlöslich sind.

Aufgrund des fehlenden Gasraums und – insbesondere bei Zylinderampullen – aufgrund des ungünstigen Verhältnisses von Durchmesser zu Länge, das sich zudem während der Anwendung beim Vorschieben des Kolbens ständig verändert, war nicht zu erwarten, daß einfach Mischkörper, wie sie von Sprühdosen bekannt sind, auch bei in übliche Arzneimittelbehältnisse gasblasenfrei gefüllten Arzneimittelsuspensionen zur Homogenisierung geeignet sein könnten.

Zudem ist von vielen Arzneistoffkristallen – insbesonder vom Insulin – bekannt, daß sie empfindlich gegenüber mechanischer Belastung sind. Rührt man z.B. eine InsulinKristallsuspension in einem Becherglas auf einem üblichen Magnetrührer, so wird ein beträchtlicher Teil der Insulinkristalle aufgrund der Einwirkung des Magnet-Rührstäbchens zerrieben. Durch die damit verbundene Oberflächenvergrößerung der Teilchen wird ihre Lösungsgeschwindigkeit und damit der Depoteffekt in unerwünschter Weise verändert. Eine solche Veränderung der Partikelgröße war auch bei Verwendung von Mischkörpern in Arzneimittelbehältnissen zu befürchten.

Es wurde nun überraschenderweise gefunden, daß auch Arzneimittelsuspensionen, vor allem gasblasenfrei abgefüllte, mit Hilfe von Mischköpern leicht in eine homogene Suspension gebracht werden können, wenn der Mischkörper inert ist, eine geeignete Dimension und eine hinreichend unterschiedliche Dichte zur Arzneimittelsuspension aufweist. Alle genannten Parameter sind hierbei durch einfache Vorversuche leicht zu ermitteln. Eine hinreichend unterschiedliche Dichte ist gegeben, wenn die Dichte der Mirshkörpers mindestens 10%, vorzugsweise mindestens 50%, insbesondere mindestens 100% höher als die des suspendierten Arzneimittels ist.

Überraschenderweise werden Insulin-Kristallsuspensionen, wie sie in den weiter hinten angeführten Beispielen Verwendung fanden, unter den beschriebenen Bedingungen praktisch nicht in ihrer Partikelgröße verändert.

Da die Arzneimittelbehälter, vor allem Spritzampullen, beispielsweise übliche Zylinderampullen oder auch Zweikammer-Spritzen (z.B. Pharm. Ind. 46 (1984, Nr. 3) 317), üblicherweise aus Glas bestehen, werden die Mischkörper vorteilhaft ebenfalls aus Glas gefertigt. Geeignet sind aber auch Kunststoffe, beispielsweise Polymere fluorierter Ethylene wie PTFE oder keramische bzw. metallische, gegebenenfalls beschichtete Körper.

Die Gestalt der Mischkörper kann durch entsprechende Vorversuche optimert werden, wobei zweckmäßigerweise darauf geachtet wird, daß das Volumen der Mischkörper relativ zum Reservoir gering bleibt, so daß vergleicheweise kleine Toträume resultieren. Im allgemeinen eignen sich einfache Formen für die Mischkörper wie Zylinder oder vorzgsweise Kugeln, aber auch Formkörper mit turbulenzenerzeugender Oberflächengestaltung wie Kugeln mit Bohrungen oder andere kompliziertere Formen kommen in Betracht.

Unter "inert" wird ein Mischkörper verstanden, der weder chemisch noch physikalisch in störender Weise mit der Arzneimittelzubereitung in Wechselwirkung tritt. Es darf also weder eine chemische Veränderung noch eine physikalische Beeinträchtigung wie Adsorption oder Abrieb in nennenswertem Maße auftreten.

Im Sinne der Erfindung geeignet ist also ein System, das mit wenigen Kipp- oder Schüttelbewegungen eine homogene Aufschüttelung erlaubt. Diese Aufgabe wird durch einen Einkompartiment-Behälter mit den Merkmelen des Anspruchs 1 gelöst.

In den folgenden Beispielen wird die Erfindung näher erläutert.

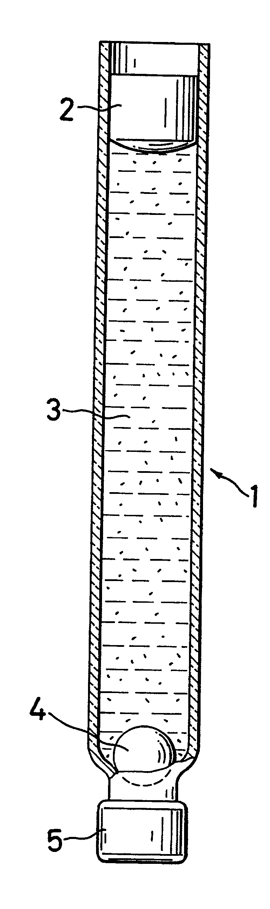

In allen Beispielen und Vergleichsbeispielen wurden die in der Figur schematisch dargestellten Zylinderampullen (1) eingesetzt, die oben einen beweglichen Kolben (2) aufweisen, auf den eine (nicht dargestellte) Kolbenstange einwirken kann. Die Ampulle ist blasenfrei mit der Arzneimittelsuspension (3) gefüllt und enthält einen oder mehrere Mischkörper (4). In der Figur ist als Mischkörper eine Kugel dargestellt. Das Auslaßstück (5) ist in bekannter Weise verschlossen.

Als Arzneimittelsuspension dient eine wäßrige Insulin-Depotzubereitung (Basal H Insulin U-100 Hoechet[R]), die pro $\mu\ell$ 0,1 I.E. Insulin enthält. Die Aufschüttelbarkeit des Sediments wurde nach zwölfstüdiger Standzeit (kopfstehend) durch Kippbewegungen von 180° visuell beurteilt.

In Vergleichsbeispiel 1 bis 3 und Beispiel 1 bis 4 hatten die Zylinderampullen (1) eine Länge von 62,3 mm und einen Innen-Durchmesser von 6,85 mm.

Vergleichsbeispiel 1 :

Kein Glasperlenzusatz
Beurteilung : Homogene Aufschüttelung durch einfache Kippbewegungen in akzeptablem Zeitraum nicht möglich.

Vergleichsbeispiel 2 :

Zusatz einer Glasperle von 3 mm Durchmesser.
Beurteilung : Aufschüttelbarkeit nicht reproduzierbar. Sediment gelangt mit einer Kugel in den Auslaßteil der Zylinderampulle und fixiert die Kugel dort.

Vergleichsbeispiel 3 :

Zusatz von 2 Glasperlen, Durchmesser 3 mm.
Beurteilung : Wie bei Vergleichabeispiel 2.

Beispiel 1 :

Zusatz einer Glasperle vom Durchmesser 4 mm.
Beurteilung : Homogene Aufachüttelung durch 6 bis 9 Kippbewegungen.

Beispiel 2 :

Zusatz von 2 Glasperlen, Durchmesser 4 mm.
Beurteilung : Wie bei Beispiel 1.

Beispiel 3 :

Zusatz einer Glasperle vom Durchmesser 5 mm.
Beurteilung : homogene Aufschüttelung durch 3 bis 4 Kipp bewegungen.

Beispiel 4 :

Zusatz einer Glasperle von 6 mm Durchmesser.
Beurteilung : Wie bei Beispeil 3.

Beispiele 5 und 6 :

Zylinderampullen von 58 mm Länge und 6,85 mm Innen-Durchmesser, die eine Glaskugel (Durchmesser 5 mm) enthielten, wurden gasblasenfrei mit der Insulin-Suspension gefüllt und in einen handelsüblichen Insulin-"Pen" eingesetzt.

Nach unterschiedlichen Standzeiten zwischen den Entnahmen wurde der Inhalt durch fünf 180°-Kippbewegungen resuspendiert und Jeweils 40 µl, entspr. 4 I.E. Insulin, in fünf Probenröhrchen dosiert. Zur Ermittlung der Homogenität wurde der Gehalt der einzelnen Proben an Insulin analytisch bestimmt.

Der Mittelwert ($\bar{x}$) von jeweils fünf Proben und die relative Standardabweichung ($s_{rel}$) sind in der folgenden Tabelle aus zwei parallelen Versuchsserien mit zwei Insulin-"Pens" zusammengefaßt :

| Standzeit vor Resus-pendierung | $\bar{x}$ (I.E.) Beispiel 5 | Beispiel 6 | $s_{rel}$ (%) Beispiel 5 | Beispiel 6 |
|---|---|---|---|---|
| 6 Stunden | 4,4 | 3,9 | 11,1 | 5,6 |
| 72 Stunden | 4,1 | 4,0 | 8,4 | 1,2 |
| 6 Stunden | 3,9 | 4,0 | 2,3 | 3,3 |
| 16 Stunden | 3,7 | 4,0 | 4,9 | 5,7 |

**Ansprüche**

1. Zylindrischer Einkompartiment -Behälter, der auf der einen Seite mit einem beweglichen Kolben (2) und auf der anderen Seite durch ein Auslaßstück (5) verschlossen ist, dadurch gekennzeichnet, daß er mit einer Medikamentensuspension (3) im wesentlichen gasblasenfrei gefüllt ist und in der Suspension einen oder mehrere Mischkörper (4) enthält, die gegenüber der Suspension inert sind, eine von der Su-spension hinreichend unterschiedliche Dichte haben, innerhalb der Vorrichtung immer frei beweglich sind und so dimensioniert sind, daß die Suspension durch wenige Schüttelbewegungen homogenisiert wird, ohne den arzneilichen Depoteffekt der Suspension negativ zu beeinflussen.

2. Vorrichtung nach Anspruch 1, dadurch gekennzeichnet, daß der oder die Mischkörper (4) kugelförmig sind.

3. Vorrichtung nach Anspruch 1 oder 2, dadurch gekennzeichnet, daß ein bis drei kugelförmige Mischkörper enthalten sind.

4. Vorrichtung nach einem oder mehreren der vorhergehenden Ansprüche, dadurch gekennzeichnet, daß die Dichte des Mischkörpers (4) mindestens 10% höher als die der Suspension (3) ist.

5. Vorrichtung nach einem oder mehreren der vorhergehenden Ansprüche, dadurch gekennzeichnet, daß die Dichte des Mischkörpers (4) mindestens 50% höher als die der Suspension (3) ist.

6. Vorrichtung nach einem oder mehreren der vorhergehenden Ansprüche, dadurch gekennzeichnet, daß die Dichte des Mischkörpers (4) mindestens 100% höher als die der Suspension ist.

7. Vorrichtung nach einem oder mehreren der vorhergehenden Ansprüche, dadurch gekennzeichnet, daß der Mischkörper (4) aus Glas, Metall, Kunststoff oder Keramik besteht.

8. Vorrichtung nach Anspruch 7, dadurch gekennzeichnet, daß der Mischkörper beschichtet ist.

9. Vorrichtung nach einem oder mehreren der vorhergehenden Ansprüche, dadurch gekennzeichnet, daß die Suspension (3) eine Kristallsuspension ist.

10. Vorrichtung nach einem oder mehreren der vorhergehenden Ansprüche, dadurch gekennzeichnet, daß die Suspension (3) eine Isulin-Kristallsuspension ist.

## Claims

1. A cylindrical single-compartment container which is sealed on one side by a movable piston (2) and on the other side by an outlet piece (5), wherein the container is filled with a medicament suspension (3) which is essentially free from gas bubbles, and contains, in the suspension, one or more mixing elements (4) which are inert toward the suspension, have a density sufficiently different from the suspension, are always freely movable within the device and have dimensions such that the suspension is homogenized by a few shaking movements without adversely affecting the medicamentous depot effect of the suspension.

2. The device as claimed in claim 1, wherein the mixing element or elements (4) are spherical.

3. The device as claimed in claim 1 or 2, containing one to three spherical mixing elements.

4. The device as claimed in one or more of the previous claims, wherein the density of the mixing element (4) is at least 10% greater than that of the suspension (3).

5. The device as claimed in one or more of the previous claims, wherein the density of the mixing element (4) is at least 50% greater than that of the suspension (3).

6. The device as claimed in one or more of the previous claims, wherein the density of the mixing element (4) is at least 100% greater than that of the suspension.

7. The device as claimed in one or more of the previous claims, wherein the mixing element (4) comprises glass, metal, plastic or ceramic.

8. The device as claimed in claim 7, wherein the mixing element is coated.

9. The device as claimed in one or more of the previous claims, wherein the suspension (3) is a crystal suspension.

10. The device as claimed in one or more of the previous claims, wherein the suspension (3) is an insulin crystal suspension.

## Revendications

1. Récipient cylindrique à un seul compartiment, qui est fermé d'un côté par un piston mobile (2) et de l'autre côté par une pièce d'évacuation (5), caractérisé en ce qu'il est rempli, sensiblement sans bulles de gaz, de médicaments en suspension (3) et contient dans la suspension un ou plusieurs corps mélangeurs (4) qui sont inertes par rapport à la suspension, qui possèdent une densité suffisamment différente de celle de la suspension, qui sont toujours librement déplaçables à l'intérieur du dispositif et qui sont dimensionnés de telle sorte que la suspension est homogénéisée par quelques agitations, sans influence négative sur l'effet de dépôt médical de la suspension.

2. Dispositif selon la revendication 1, caractérisé en ce que le ou les corps mélangeurs (4) sont sphériques.

3. Dispositif selon la revendication 1 ou 2, caractérisé en ce qu'il contient un à trois corps mélangeurs sphériques.

4. Dispositif selon une ou plusieurs des revendications précédentes, caractérisé en ce que la densité du corps mélangeur (4) est supérieure d'au moins 10% à celle de la suspension (3).

5. Dispositif selon une ou plusieurs des revendications précédentes, caractérisé en ce que la densité du corps mélangeur (4) est supérieure d'au moins 50% à celle de la suspension (3).

6. Dispositif selon une ou plusieurs des revendications précédentes, caractérisé en ce que la densité du corps mélangeur (4) est supérieure d'au moins 100% à celle de la suspension.

7. Dispositif selon une ou plusieurs des revendications précédentes, caractérisé en ce que le corps mélangeur (4) est constitué de verre, de métal, de matière plastique ou de matériau céramique.

8. Dispositif selon la revendication 7, caractérisé en ce que le corps mélangeur est pourvu d'une enduction.

9. Dispositif selon une ou plusieurs des revendications précédentes, caractérisé en ce que la suspension (3) est une suspension cristalline.

10. Dispositif selon une ou plusieurs des revendications précédentes, caractérisé en ce que la suspension (3) est une suspension cristalline d'insuline.